# EUROPÄISCHE PATENTANMELDUNG

(11) **EP 0 597 435 A1**
(43) Veröffentlichungstag der Anmeldung: **18.05.1994**
(21) Anmeldenummer: 93118106.9
(22) Anmeldetag: 09.11.1993
(51) Int. Cl.: A61L 2/04

(54) **Pasteurisierung von Hemoglobin**

(30) Priorität: 11.11.1992 DE 4237977
(71) Anmelder: B. BRAUN MELSUNGEN AG, D-34212 Melsungen (DE)
(72) Erfinder: Zippel, Matthias, D-34286 Spangenberg (DE); Heuer, Yvonne, D-34212 Melsungen (DE); Messing, Falk, D-34132 Kassel (DE)
(74) Vertreter: Jönsson, Hans-Peter, Dr.Dipl.-Chem.

(57) **Zusammenfassung**

Gegenstand der Erfindung ist ein Verfahren zur Pasteurisierung von Hemoglobin, das so erhältliche bakterienfreie, pilzfreie und virusfreie Hemoglobin sowie die Verwendung des so hergestellten Hemoglobins als Basiskomponente für einen Blutersatz.

Das Verfahren zur Pasteurisierung von Hemoglobin wird dadurch realisiert, daß durch Erhitzen unter Verminderung der biologischen Aktivität von Viren bei im wesentlichen Aufrechterhalten der biologischen Aktivität von Hemoglobin während der Pasteurisierung ein nicht reduzierender Stabilisator für das Hemoglobin eingesetzt wird. Vorzugsweise ist dieser Stabilisator ausgewählt aus Zuckeralkoholen und Polyglycerinen.

## Beschreibung

Gegenstand der Erfindung ist ein Verfahren zur Pasteurisierung von Hemoglobin.

Hemoglobin menschlichen oder tierischen Ursprungs ist nach entsprechender Reinigung und chemischer Modifizierung eine naheliegende und vielversprechende Basiskomponente für einen Blutersatz (vgl. z. B. W. J. Dodds: "Blood Substitutes" in Adv. Vet. Sci. Comp. Med. 36, 257 bis 290 (1991). Verfahren zur Reinigung und Modifizierung von Hemoglobin sind beispielsweise in der EP-A-0 277 289 beschrieben. Entsprechende Produkte zeigen vielversprechende Eigenschaften in präklinischen Untersuchungen.

Die parenterale Anwendung eines derartigen Produktes bedingt selbstverständlich die Abwesenheit von potentiell infektiösen Agentien, d. h. die Freiheit von Parasiten, Bakterien, Pilzen und Viren. Entsprechende Produkte müssen daher einem Dekontaminationsverfahren unterzogen werden. Wegen der hohen Empfindlichkeit des Hemoglobin-Moleküls gegen Denaturierung und Oxidation können Hemoglobin-Lösungen jedoch nicht mit der üblichen Hitzesterilisation dekontaminiert werden. Statt dessen werden Hemoglobin-Lösungen, wie andere empfindliche Proteine auch, üblicherweise durch Sterilfiltration entkeimt. Dieses Verfahren ist für die Entfernung von Bakterien und Pilzen ein sicherer und validierter Standard. Es versagt jedoch bei der Anwesenheit von Viren, so daß zur Sicherung der Freiheit von Viruskontaminationen weitere spezielle Verfahren angewendet werden müssen.

Im Stand der Technik sind beispielsweise folgende Verfahren bekannt, die für empfindliche Proteine anwendbar sein sollen:
So wird in Horowitz et al, Transfusion 25, 523 - 527 (1985) die Pasteurisierung von Antihemophiliefaktoren (AHF) bei Temperaturen im Bereich von 60° C im Verlauf von 10 Stunden beschrieben. Auch ist die Behandlung von Proteinen mit Licht oder UV-Strahlung beispielsweise aus J. L. Matthews et al, Transfusion 31, 636 - 641 (1991) bekannt. Aus Horowitz et al, Transfusion 25, 516 - 522 (1985) ist weiterhin die Behandlung mit Lösemittel-Tensid-Gemischen bekannt.

Jedes der drei bekannten Verfahren hat die ihm eigenen Vorteile und Nachteile. Die größte Sicherheit zur Herstellung von virusfreien Präparaten scheint das Pasteurisierungsverfahren zu geben, da hierbei auch unbehüllte Viren inaktiviert werden können. Wegen ihrer hohen Widerstandsfähigkeit werden diese Viren durch Bestrahlung mit sichtbarem Licht oder UV-Strahlung nur bedingt und durch die Lösemittel-Tensid-Behandlung überhaupt nicht beeinflußt.

Die Pasteurisierung von Proteinen ist jedoch nicht ohne weiteres auf die Pasteurisierung von Hemoglobin zu übertragen. Hemoglobin liegt bekanntermaßen als labiler Komplex aus 4 Hemoglobin-Untereinheiten vor, die jeweils ein Sauerstoffmolekül tragen. Unter Temperaturbelastung erfolgt sehr rasch die Oxidation des zentralen Eisenatoms von Eisen-II zu Eisen-III (biologisch inaktives Met-Hemoglobin). Met-Hemoglobin (Met-Hb) ist thermisch labiler als normales Hemoglobin und unterliegt sehr leicht irreversiblen Denaturierungsprozessen. Somit geht bei normaler Pasteurisierung ein großer Teil des Hemoglobins durch Umwandlung zu Met-Hb und nachfolgender Denaturierung verloren.

Bekanntermaßen gelingt jedoch die Pasteurisierung von Hemoglobin, wenn der Sauerstoff aus dem Molekül vorher entfernt wird. Dies gelingt beispielsweise durch Verdrängung des Sauerstoffs mittels eines starken Komplexbildners, wie Kohlenmonoxid, durch chemische Reduktion oder physikalische Deoxygenierung (Stripping mit Inertgas). In sauerstoff-freier Form unterbleibt die Umwandlung zu Met-Hb bei erhöhter Temperatur und die nachfolgende Denaturierung. Im Stand der Technik sind beispielsweise die Pasteurisierung von Kohlenmonoxid-Hemoglobin (CO-Hb) (EP 0290252) und von sauerstoff-freiem Hemoglobin in Gegenwart eines Reduktionsmittels bekannt (WO 85/04407 entspricht EP-A-0 179 075).

Die Pasteurisierung von CO-Hb ist wegen der hohen Affinität des Kohlenmonoxids zu Hemoglobin in Bezug auf die Denaturierung sehr vorteilhaft, da Restmengen Sauerstoff in der Lösung des Hemoglobins nicht stören können. Die Affinität des Kohlenmonoxids zu Hemoglobin ist bekanntermaßen so hoch, daß das Kohlenmonoxid durch Sauerstoff nicht verdrängt werden kann, wodurch ein absoluter Sauerstoffausschluß beim Pasteurisierungsverfahren nicht erforderlich ist. Bei der weiteren Aufbereitung des Hemoglobins tritt jedoch der entscheidende Nachteil auf, daß Kohlenmonoxid das Hemoglobin vollständig für die vorgesehene Anwendung als Blutersatz inaktiviert, so daß nach der Pasteurisierung in einem aufwendigen, sehr komplexen Prozeß das Kohlenmonoxid wieder aus dem Komplex entfernt werden muß.

Die Pasteurisierung von Sauerstoff-freiem Hemoglobin in Abwesenheit von Kohlenmonoxid ist nur bei absolutem Sauerstoffausschluß möglich. Bereits Restmengen von Sauerstoff binden sich sofort an ein Hemoglobin-Molekül, das unter den Pasteurisierungsbedingungen rasch Met-Hb bildet, den Sauerstoff an ein weiteres Hemoglobin-Molekül weitergibt und selber denaturiert. Auf diese Art und Weise wird nach kurzer Zeit das Hemoglobin zu einem erheblichen Anteil denaturiert.

Dieser absolute Sauerstoff-Ausschluß ist technisch nicht realisierbar, so daß, wie oben angeführt, die Pasteurisierung in Anwesenheit eines Reduktionsmittels, das auch letzte Spuren von Sauerstoff eliminiert, durchgeführt worden ist. Dieses Verfahren hat jedoch den prinzipiellen Nachteil, daß das Reduktionsmittel selbst wegen seiner chemischen Reaktivität unter den Pasteurisierungsbedingungen mit der Proteinkomponente des Hemoglobins reagieren kann. Darüberhinaus müssen die eingesetzten Reduktionsmittel, beispielsweise reduzierende Farbstoffe, Sulfoxyverbindungen oder Mercaptane nach dem Pasteurisieren möglichst vollständig aus den Produkten entfernt werden, da Restmengen dieser Reduktionsmittel sonst nach dem Pasteurisierungsprozeß bei der klinischen Anwendung des Produktes zu Unverträglichkeitsreaktionen bei den Patienten führen können. Deshalb müssen nach dem Pasteurisierungsprozeß in einem aufwendigen Verfahren nach derartigen Verfahren hergestellte Produkte gereinigt werden.

Demgemäß besteht also weiterhin ein Bedarf an einem Pasteurisierungsverfahren für Hemoglobin, das folgende Grundbedürfnisse erfüllen sollte:
- das Hemoglobin darf nicht gegen Sauerstoff inaktiviert werden
- der Pasteurisierungsprozeß soll möglichst ohne reaktive Agentien auskommen, die mit Hemoglobin reagieren und
- ein zugesetztes Agens soll in Restmengen physiologisch unbedenklich sein.

Überraschenderweise wurde gefunden, daß das vorgenannte Verfahren zur Pasteurisierung von Hemoglobin dann erfolgreich durchgeführt werden kann, wenn dem Hemoglobin ein nicht reduzierender Stabilisator zugesetzt wird.

Eine erste Ausführungsform der vorliegenden Erfindung betrifft somit ein Verfahren zur Pasteurisierung von Hemoglobin durch Erhitzen unter Verminderung der biologischen Aktivität von Viren, bei im wesentlichen Aufrechterhalten der biologischen Aktivität von Hemoglobin, das dadurch gekennzeichnet ist, daß man während der Pasteurisierung einen nicht reduzierenden Stabilisator für das Hemoglobin einsetzt.

Während also bisher im Stand der Technik die Inaktivierung und die damit verbundene Denaturierung des Hemoglobins durch Anwendung niedriger Temperaturen oder Zugabe von reduktiven Stabilisierungsmitteln verhindert werden konnte, ist es mit Hilfe der vorliegenden Erfindung erstmals möglich, ein Verfahren zur Pasteurisierung von Hemoglobin zur Verfügung zu stellen, bei dem das Hemoglobinmolekül nicht auf chemischem Wegen gegen Sauerstoff inaktiviert wird und das Pasteurisierungsverfahren insbesondere ohne reaktive Reagentien auskommt, die mit Hemoglobin reagieren.

Überraschenderweise wurde gefunden, daß insbesondere Zuckeralkohole und Polyglycerine zur Stabilierung des Hemoglobins besonders geeignet sind. Diese Substanzen sind als mehrwertige Alkohole gegenüber Hemoglobin unter den Pasteurisierungsbedingungen chemisch inert und darüberhinaus physiologisch unbedenklich. Bei Anwesenheit dieser Stabilisatoren ließen sich deoxygenierte Hemoglobin-Lösungen nahezu ohne Denaturierung pasteurisieren, ohne daß der Zusatz eines Reduktionsmittel erforderlich wäre. Auch Restmengen an Sauerstoff, die während der Pasteurisierung in dem Reaktionssystem vorhanden gewesen sind, erwiesen sich als nicht störend.

Demgegenüber wurde festgestellt, daß bei der Pasteurisierung von deoxygeniertem Hemoglobin alleine in wäßriger Lösung ebenso eine starke Denaturierung beobachtet wurde, wie in dem Falle, daß der wäßrigen Lösung als nichtreduzierender Stabilisator Saccharose beigesetzt wurde. Beide Systeme sind somit keinesfalls geeignet, ein hochaktives, jedoch virusfreies Hemoglobin zur Verfügung zu stellen.

Wie die durchgeführten Versuche zeigen, ist die Auswahl der Zuckeralkohole nicht von besonders kritischer Bedeutung. Vielmehr lassen sich nach dem derzeitigen Kenntnisstand sämtliche bekannten Zuckeralkohole im Sinne der vorliegenden Erfindung einsetzen, die nach "Römpp Chemie Lexikon", 9. Aufl., S. 5165 unter die Gruppenbezeichnung für die aus Monosacchariden durch Reduktion der Carbonyl-Funktion entstehenden Polyhydroxyverbindungen fallen, die keine Zucker sind, gleichwohl aber süß schmecken und deshalb gegebenenfalls Verwendung als Zuckeraustauschstoffe finden können. In diesem Sinne sind die Zuckeralkohole gemäß der vorliegenden Erfindung vorzugsweise ausgewählt aus Tetriten, Pentiten und Hexiten. Besonders bevorzugt im Sinne der vorliegenden Erfindung sind die natürlich vorkommenden Zuckeralkohole, insbesondere Glycerin, Threit und Erythrit, Adonit (Ribit), Arabit (früher: Lyxit) und Xylit, Dulcit (Galactit), Mannit und Sorbit (Glucit). Besonders bevorzugt im Sinne der vorliegenden Erfindung sind Glycerin und Sorbit, da diese Stabilisatoren bezüglich des toxikologischen Potentials eventueller Restmengen in Verbindung mit der parenteralen Ernährung besonders gut erforscht sind, obwohl sämtliche der vorgenannten Zuckeralkohole im Sinne der vorliegenden Erfindung vorteilhafterweise eingesetzt werden können.

Im gleichen Sinne wie die oben genannten Zuckeralkohole sind anstelle von Glycerin auch deren Oligomere, die sogenannten Polyglycerine, als nichtreduktiver Stabilisator für das Hemoglobin einsetzbar. Bekanntermaßen leiten sich die Polyglycerine von dem Monomer Glycerin ab. Polyglycerine sind beispielsweise in "Ullmann's Encyclopedia of Industrial Chemistry", 5. Aufl. Bd. A12, S. 487/488 als Ether von Glycerin definiert, die auch in technischem Maße durch basenkatalysierte Kondensation von Glycerin sowie als Nebenprodukt bei der Epichlorhydrinhydrolyse erhalten werden. Die Polyglycerine haben relative Molekülmassen von 166 (6-C-Atome) bis 2238 (90-C-Atome) und hier bis 32 Hydroxylgruppen. Bekanntermaßen sind diese Verbindungen eßbar und werden als Veresterungskomponenten benutzt. Besondere Bedeutung haben die kurzkettigen Polyglycerine, insbesondere das Diglycerin und das Triglycerin.

Die im Sinne der Erfindung einzusetzenden Stabilisatoren werden üblicherweise in einer Menge eingesetzt, die ausreichend ist, die Stabilisierungswirkung für das Hemoglobin zu entfalten. Wird jedoch das Verhältnis von Hemoglobin zu Stabilisator zu klein gewählt, d. h. der Stabilisator in einem zu hohen Überschuß eingesetzt, so ist zwar die Stabilisierungswirkung vorhanden, jedoch wird das Verfahren aufgrund des hohen Lösungsmitteleinsatzes unwirtschaftlich, da nur eine geringe Menge an Hemoglobin pasteurisiert wird. Andererseits ist es selbstverständlich erforderlich, eine gewisse Mindestmenge an Stabilisator einzusetzen. Diese Mindestmenge an Stabilisator richtet sich zum einen nach dem jeweils eingesetzten Stabilisator, so daß unterschiedliche Stabilisatoren auch unterschiedliche Mindestmengen erfordern können. Zur Gewährleistung einer ausreichenden Stabilisierungswirkung hat es sich jedoch herausgestellt, daß das Gewichtsverhältnis von Hemoglobin zu Stabilisator im Sinne der vorliegenden Erfindung kleiner als 1, vorzugsweise kleiner als 0,5, sein sollte. Dies bedeutet, daß der Stabilisator in einem Überschuß gegenüber dem Hemoglobin während der Pasteurisierung vorhanden sein sollte. Da es sich bei der Stabilisierung gemäß der vorliegenden Erfindung nicht um eine chemische Stabilisierung durch Bindung des Glycerinmoleküls an Hemoglobin handelt, ist hierbei nicht von "stöchiometrischen" Verhältnissen auszugehen, vielmehr sind Gewichtsverhältnisse von Hemoglobin zu Stabilisatoren in ihrer Bedeutung zu beachten.

Hemoglobin kommt bekanntermaßen in verdünnten wäßrigen Lösungen als auch in trockener Form in den Handel. Besonders bevorzugt im Sinne der vorliegenden Erfindung ist der Einsatz von Hemoglobin in einer 5 bis 50 Gew.-%igen Lösung, insbesondere in einer Lösung, die 5 Gew.-% bis 20 Gew.-% Hemoglobin enthält. Obwohl das Lösungsmittel des Hemoglobins während der Pasteurisierung nur eine untergeordnete Bedeutung hat, ist es im Sinne der vorliegenden Erfindung, Hemoglobin in Form einer entsprechenden wäßrigen Lösung einzusetzen. Diese wäßrige Lösung hat gleichermaßen den Vorteil, daß neben dem Hemoglobin auch der Stabilisator gelöst werden kann. Dementsprechend kommt es bei der Auswahl der Stabilisatoren im Pasteurisierungsverfahren in der Regel nicht auf den Aggregatzustand des Stabilisators bei Raumtemperatur an. So können beispielsweise feste Stabilisatoren, beispielsweise Sorbit, in gleicher Weise wie flüssige Stabilisatoren, beispielsweise Glycerin, unter den oben genannten Bedingungen eingesetzt werden. Obwohl die Pasteurisierung von Hemoglobin in wäßriger Lösung besonders bevorzugt ist, da Hemoglobin in dieser Form im Handel erhältlich ist, ist es jedoch in gleicher Weise prinzipiell möglich, Hemoglobin auch in Form fester Substanzgemische unter Zusatz der erfindungsgemäßen Stabilisatoren zu pasteurisieren.

Derartige Pasteurisierungsbedingungen sind im Stand der Technik bei der Pasteurisierung von Hemoglobin unter reduktiven Bedingungen bekannt. So ist aus der WO 85/044 07 eine Pasteurisierungszeit von 5 bis 15 Stunden im Falle der Anwendung wäßriger Lösung von Hemoglobin besonders bevorzugt. Für den Fall, daß die Pasteurisierung in trockener, wasserfreier Form durchgeführt wird, ist auch im Sinne der vorliegenden Erfindung eine Pasteurisierungszeit von 20 bis 96 Stunden bevorzugt. Die Pasteurisierungsbedingungen gemäß der vorliegenden Erfindung umfassen weiterhin einen Temperaturbereich von etwa 50° bis 80° C, wobei die Pasteurisierungstemperatur von 60° C besonders bevorzugt wird. Ursache hierfür ist ein besonders ausgewogenes Verhältnis von Reaktionsdauer, Erhalt der chemischen Aktivität des Hemoglobins und gleichzeitiger guter Verminderung der Aktivität von Viren.
Die Anwesenheit von im Stand der Technik bekannten Reduktionsmitteln und deren Mengen, beispielsweise reduzierende Farbstoffe, Sulfoxyverbindungen oder Mercaptane, insbesondere Dithionite, Metabisulfite, Sulfite, reduzierte Glutathione und Dithiothreitole während der Pasteurisierung gemäß der vorliegenden Erfindung stören in der Regel nicht. So ist beispielsweise die Verwendung von N-Acetylcystein besonders bevorzugt. Dementsprechend besteht eine weitere Ausführungsform der vorliegenden Erfindung darin, daß man während der Pasteurisierung die genannten Reduktionsmittel zusetzt. Hierbei ist es jedoch erforderlich, in einem Aufbereitungsschritt Restmengen dieser Reduktionsmittel möglichst vollständig zu entfernen.

Nach der Durchführung der Pasteurisierung, d. h. im Anschluß an das Erhitzen des Reaktionsguts ist dann eine Abtrennung des Stabilisators von dem Hemoglobin erforderlich. Üblicherweise wird diese Abtrennung bei niedriger Temperatur, insbesondere Raumtemperatur, durchgeführt. Hierzu wird in der Regel das Reaktionsgut zunächst auf Raumtemperatur abgekühlt. In einer besonderen Ausführungsform der vorliegenden Erfindung wird dann der Stabilisator durch Dialyse entfernt. Im Stand der Technik sind eine Reihe von Dialyseverfahren und Vorrichtungen zur Entfernung von einzelnen Bestandteilen aus Gemischen bekannt, die dem Fachmann auf dem hier vorliegenden Gebiet geläufig sind.

In einer besonders bevorzugten Ausführungsform der vorliegenden Erfindung wird die Dialyse in Form der Tangentialflußfiltration durchgeführt. Beispielsweise kann unter Verwendung eines Cutoff-Filters für Molekulargewichte von 10 000 unter Volumenkonstanthaltung, d. h. durch Nachfüllen von Wasser in dem System eine außerordentlich lange Standzeit erhalten werden. Die Tangentialflußfiltration (auch cross-flow-filtration genannt) kann bekanntermaßen kontinuierlich durchgeführt werden. Gemäß der vorliegenden Erfindung werden die Stabilisatoren, insbesondere Zuckeralkohole und Polyglycerine somit von dem Hemoglobin abgetrennt.

Zur Entfernung von Bakterien oder Pilzen, ist es weiterhin möglich, vor dem erfindungsgemäßen Schritt der Pasteurisierung, während dieses Pasteurisierungsschrittes oder im Anschluß daran eine an sich bekannte Sterilfiltration durchzuführen. Mit Hilfe dieser Sterilfiltration werden Bakterien und Pilze sicher aus dem System entfernt.

Mit Hilfe des oben genannten Verfahrens ist ein bakterienfreies, pilzfreies und virenfreies Hemoglobin erhältlich. Es wurde gefunden, daß in dem so hergestellten Hemoglobin nur ein äußerst geringer Anteil denaturierte. Der Grad der Denaturierung wurde durch Trübungsmessungen von pasteurisierten Hemoglobinlösungen bestimmt. Weil mit üblichen naß-chemischen Analyseverfahren eine Denaturierung der Proteinkomponente des Hemoglobins nicht zweifelsfrei nachgewiesen werden konnte, wurde mit Hilfe eines Streulichtfotometers die Trübung von Hemoglobinlösungen bestimmt. Das Ausmaß der Trübung gibt hierbei Auskunft über die Denaturierung nach der Pasteurisierung. Darüberhinaus wurden Untersuchungen mittels isoelektrischer Fokusierung durchgeführt, um zu belegen, daß das Hemoglobinmolekül während der Pasteurisierung und der anschließenden Aufbereitung nicht chemisch verändert wurde.

Die nachfolgenden Beispiele erläutern die vorliegende Erfindung.

### Beispiele

### Beispiel 1

Es wurde ein 50:50 (Vol.%) Gemisch einer partiell durch Begasung mit Stickstoff deoxygenierten Hemoglobinlösung in Wasser mit einem aliquoten Volumen Glycerin gemischt. Die eingesetzte Hemoglobinlösung enthielt 8,3 Gew.-% bovines Hemoglobin mit einem MetHb-Anteil von 2,9 % und einer Sauerstoffsättigung von 15 %. Die Versuchslösung war daher wie folgt charakterisiert: der Gehalt an HbA betrug 6,2 % und der Gehalt an Met-Hb 4,8 %. Diese Lösung wurde 10 Stunden auf 60° C unter Sauerstoffabschluß erhitzt.

Nach dem Abkühlen auf Raumtemperatur wurde die Versuchslösung analytisch untersucht. Das Ausmaß der bei der Pasteurisierung teilweise stattfindenden Denaturierung wurde durch Bestimmung des wasserunlöslich gewordenen Proteinanteils über die Trübung der Lösung (1:10 mit Wasser verdünnt) nach an sich bekannten Verfahren der Bestimmung von trüben Lösungen bestimmt.

Die Tabelle 1 gibt dabei die Trübungen vor der Pasteurisierung und nach der Pasteurisierung wieder. Die Trübung ist jeweils in Trübungseinheiten bezogen auf Formazan wiedergegeben (TE/F).

### Beispiel 2

Analog Beispiel 1 wurde unter vollständiger Substitution des Glycerins durch eine entsprechende Menge Sorbit eine Versuchslösung erhalten, die 6,2 % HbA mit 4,8 % Met-Hb enthielt. Die Ergebnisse der Pasteurisierung sind in der Tabelle 1 benannt.

### Vergleichsbeispiel 1

Unter vollständiger Substitution des Glycerins gemäß Beispiel 1 durch Wasser wurde eine Versuchslösung erhalten, die 4,1 % HbA mit 3,6 % Met-Hb enthielt. Die Trübungsmessungen im Anschluß an die Pasteurisierung sind in Tabelle 1 wiedergegeben.

### Vergleichsbeispiel 2

Analog Beispiel 1 unter vollständiger Substitution des Glycerins durch 1,2-Propylenglycol wurde eine Versuchslösung erhalten, die 4,3 % HbA mit 13,3 % Met-Hb enthielt. Die Trübungen der Lösungen (1:10 verdünnt, Angabe in Trübungseinheiten Formazan (TE/F)) vor und nach der Pasteurisierung sind in der nachfolgenden Tabelle 1 genannt.

**Tabelle 1**

| Beispiel | Trübung (TE/F) | |
|---|---|---|
| | vorher | nachher |
| 1 | 2,3 | 13,3 |
| 2 | 4,0 | 15,0 |
| Vgl. 1 | 2,2 | 53 |
| Vgl. 2 | 130 | 165 |

Aus der Tabelle 1 ist erkennbar, daß der Zusatz von Glycerin und Sorbit als Stabilisator im Sinne der vorliegenden Erfindung eine deutlich stabilisierende Wirkung hat. Das chemisch nah verwandte 1,2 Propylenglycol destabilisiert jedoch Hemoglobin bereits bei Raumtemperatur, wodurch sich ein erhöhter Trübungsgrad bereits vor der Pasteurisierung bemerkbar macht.

### Beispiel 3

Unter Verwendung der oben genannten Hemoglobinlösung (8,3 Gew.-%ig) und Glycerin in jeweils verschiedenen Mengen wurde die Abhängigkeit der Denaturierung von der zugesetzten Menge an Stabilisator untersucht. Aus der nachfolgend genannten Tabelle 2 gehen entsprechende Lösungen hervor, die unter Verwendung der dort genannten Bestandteile jeweils 10 Stunden bei 60° C pasteurisiert wurden. Das Ausmaß der über die Trübung der Lösung ermittelten Denaturierung wird ebenfalls in dieser Tabelle wiedergegeben.

**Tabelle 2**

| Beispiel | Hemoglobin (Vol%) | Glycerin (Vol%) | HbA* (%) | Met-Hb* (%) | Trübung** (TE/F) | Trübung (normiert)*** |
|---|---|---|---|---|---|---|
| 3.1 | 50 | 50 | 5,3 | 9,0 | 13,3 | 26 |
| 3.2 | 60 | 40 | 5,8 | 9,7 | 30 | 52 |
| 3.3 | 70 | 30 | 6,3 | 8,3 | 48 | 76 |
| 3.4 | 80 | 20 | 7,0 | 8,2 | 54 | 77 |
| 3.5 | 90 | 10 | 7,6 | 6,6 | 67 | 88 |
| Vgl.3 | 100 | 0 | 8,3 | 2,9 | 65 | 78 |

| | | | | | | |
|---|---|---|---|---|---|---|
| * vor der Pasteurisierung | | | | | | |
| ** nach der Pasteurisirung | | | | | | |
| *** normiert auf Hb-Gehalt von 10% | | | | | | |

Aus dieser Tabelle ist erkennbar, daß mit steigender Glycerinmenge die Denaturierung zurückgedrängt wird. Darüberhinaus wurden sensitive analytische Untersuchungen der pasteurisierten Proben durch isoelektrische Fokussierung durchgeführt. Hierbei wurde gefunden, daß das Hemoglobinmolekül durch die Pasteurisierung nicht verändert wurde. Eine chemische Reaktion mit Glycerin fand nicht statt.

## Patentansprüche

1. Verfahren zur Pasteurisierung von Hemoglobin durch Erhitzen unter Verminderung der biologischen Aktivität von Viren bei im wesentlichen Aufrechterhalten der biologischen Aktivität von Hemoglobin, dadurch gekennzeichnet, daß man während der Pasteurisierung einen nicht reduzierenden Stabilisator für das Hemoglobin einsetzt.

2. Verfahren nach Anspruch 1, dadurch gekennzeichnet, daß der Stabilisator ausgewählt ist aus Zuckeralkoholen und Polyglycerinen.

3. Verfahren nach Anspruch 1 oder 2, dadurch gekennzeichnet, daß die Zuckeralkohole ausgewählt sind aus Tetriten, Pentiten oder Hexiten, insbesondere Glycerin, Threit, Erythrit, Adonit, Arabit, Xylit, Dulcit, Mannit und Sorbit.

4. Verfahren nach Anspruch 2, dadurch gekennzeichnet, daß die Polyglycerine Molekulargewichte im Bereich von 166 bis 2238 aufweisen.

5. Verfahren nach einem oder mehreren der Ansprüche 1 bis 4, dadurch gekennzeichnet, daß das Gewichtsverhältnis von Hemoglobin zu Stabilisator kleiner als 1, vorzugsweise kleiner als 0,5 ist.

6. Verfahren nach einem oder mehreren der Ansprüche 1 bis 5, dadurch gekennzeichnet, daß man Hemoglobin in einer 5 bis 50 Gew.-%igen, insbesondere 5 bis 20 Gew.-%igen wäßrigen Lösung einsetzt.

7. Verfahren nach einem oder mehreren der Ansprüche 1 bis 6, dadurch gekennzeichnet, daß man im Verlauf von 5 bis 15 Stunden im Falle einer wäßrigen Lösung von Hemoglobin und 20 bis 96 Stunden im Falle von trockenen, wasserfreien Zusammensetzungen erhitzt.

8. Verfahren nach einem oder mehreren der Ansprüche 1 bis 7, dadurch gekennzeichnet, daß man auf eine Temperatur von 50° C bis 80° C, insbesondere 60° C erhitzt.

9. Verfahren nach einem oder mehreren der Ansprüche 1 bis 8, dadurch gekennzeichnet, daß man ein Reduktionsmittel zusetzt, insbesondere ausgewählt aus reduzierenden Farbstoffen, Sulfoxyverbindungen, Mercaptanen, vorzugsweise Dithioniten, Metabisulfiten, Sulfiten, reduzierten Glutathionen und Dithiothreitolen.

10. Verfahren nach einem oder mehreren der Ansprüche 1 bis 9, dadurch gekennzeichnet, daß man im Anschluß an das Erhitzen das Reaktionsgut gegebenenfalls auf Raumtemperatur abkühlt und weiterhin den Stabilisator durch Dialyse entfernt.

11. Verfahren nach Anspruch 10, dadurch gekennzeichnet, daß man die Dialyse als Tangentialflußfiltration durchführt.

12. Verfahren nach einem oder mehreren der Ansprüche 1 bis 11, dadurch gekennzeichnet, daß man vor, während oder nach dem Erhitzen eine Sterilfiltration durchführt.

13. Bakterienfreies, pilzfreies und virusfreies Hemoglobin, erhältlich nach einem Verfahren wie in einem oder mehreren der Ansprüche 1 bis 12 definiert.

14. Verwendung des Hemoglobins wie in Anspruch 13 definiert, als Basiskomponente für einen Blutersatz.
